# EUROPÄISCHE PATENTSCHRIFT

(11) **EP 3 154 604 B1**
(45) Veröffentlichungstag und Bekanntmachung des Hinweises auf die Patenterteilung: **01.01.2020**
(21) Anmeldenummer: 15730096.3
(22) Anmeldetag: 10.06.2015
(51) Int. Cl.: A61M 1/10, A61M 1/12

(54) **UMPE ZUR IMPLANTIERUNG IN EIN GEFÄSS**
PUMP WHICH IS TO BE IMPLANTED INTO A VESSEL
POMPE DESTINÉE À ÊTRE IMPLANTÉE DANS UN VAISSEAU

(30) Priorität: 12.06.2014 DE 102014211216
(43) Veröffentlichungstag der Anmeldung: 19.04.2017
(73) Patentinhaber: Universität Duisburg-Essen, 45141 Essen (DE)
(72) Erfinder: NEUMANN, Till, 44869 Bochum (DE)
(74) Vertreter: RCD Patent Giesen, Schmelcher & Griebel Patentwanwälte PartG mbB
(86) Internationale Anmeldenummer: PCT/EP2015/062919
(87) Internationale Veröffentlichungsnummer: WO 2015/189259

(56) Entgegenhaltungen:
- WO-A1-2015/052303
- US-A1- 2013 138 205
- US-A1- 2014 051 908
- US-B1- 8 690 749

## Beschreibung

### Hintergrund

Herzinsuffizienz ist eine der häufigsten internistischen Krankheiten in den entwickelten Staaten dieser Welt. Dabei ist mit zunehmendem Alter eine vermehrte Häufigkeit der Herzinsuffizienz festzustellen.

Im Allgemeinen wird unter einer Herzinsuffizienz das Unvermögen des Herzens verstanden, den betroffenen Organismus mit einer ausreichenden Blutzirkulation zu versorgen. Dabei ist festzustellen, dass die verminderte Blutzirkulation (auch als vermindertes Herzzeitvolumen - HZV - bezeichnet) zu einer Mangelversorgung des Gewebes mit Sauerstoff und/oder Nährstoffen führt. Symptomatisch kann sich dies in einer verminderten körperlichen Belastbarkeit und/oder vermehrter Atemnot (sogenannte Dyspnoe) als auch verstärkter Wassereinlagerung (z.B. Beinödem) manifestieren. Auf die Ursachen soll hierbei nicht weiter eingegangen werden.

Weiterhin ist festzustellen, dass die Herzinsuffizienz auch häufig mit einem dilatierten linken Ventrikel mit reduzierter Pumpfunktion einhergeht.

Da die Bevölkerungen zunehmend älter werden ist auch mit einer zunehmenden Anzahl von Erkrankten auszugehen. Insofern ist mit einem zunehmenden Behandlungsbedarf zu rechnen.

Bisherige Behandlungsansätze orientieren sich am jeweiligen Stadium der Erkrankung.

In den sogenannten leichten und mittleren Stadien (New York Heart Association NYHA I-II) wird in der Regel medikamentös therapiert. Zudem besteht die Möglichkeit der Implantierung eines Defibrillators (implantierbarer Kardioverter-Defibrillator (ICD)) oder eine Resynchronisierungs-Therapie (CRT).

In den fortgeschrittenen als auch terminalen Stadien (New York Heart Association NYHA III-IV) ist unter Umständen eine Herztransplantation zu erwägen. Alternativ ist - insbesondere bei älteren Patienten - auch der Einsatz eines kardialen linksventrikulären Unterstützungssystems (LVAD) angezeigt. Da die Anzahl der Spenderorgane limitiert ist, ist der Einsatz eines linksventrikulären Unterstützungssystems auch als Überbrückungsmaßnahme anzutreffen.

Bisherige LVAD-Systeme bestanden aus kontaktierenden / pulsierenden Einheiten, welche teilweise - z.B. auf Grund der Größe - außerhalb des Körpers angeordnet sind. Neuere LVAD-System, sind in der Regel intra-korporal platziert und weisen einen kontinuierlichen Fluss auf. Dabei kann mit den neueren Systemen eine Förderleistung von bis zu 8 Litern in der Minute zur Verfügung gestellt werden, wodurch auch die komplette Herzleistung ersetzt werden kann.

Allerdings sind auch die verschiedenen neueren LVAD-Systeme dennoch aus den verschiedensten Gründen nachteilig, wie im Folgenden kurz dargestellt werden soll.

Allen diesen Systemen ist gemein, dass sie mittels einer Operation implantiert werden müssen. Dabei ist in aller Regel der Brustkorb zu eröffnen und häufig bedarf es der Unterstützung durch eine Herz-Lungen-Maschine. D.h. es ist ein aufwändiger und risikoreicher Eingriff in Vollnarkose nötig. Zudem weisen alle diese neueren Systeme eine externe Energieeinheit und/oder eine externe Steuereinheit auf. Dabei ist die Anbindung der externen Systeme kritisch, denn entlang der drahtgebundenen Verbindung besteht die Gefahr, dass Keime eindringen. Weiterhin sind die bisherigen neueren Systeme auf eine kontinuierliche Unterstützung ausgelegt, sodass auch in Zeiten, in denen kein erhöhter Bedarf besteht, z.B. in Ruhephasen, ein hoher Blutfluss aufrecht erhalten wird. Dies wirkt sich in aller Regel negativ auf die Größe der notwendigen Energieversorgungseinheit aus und steht daher einer intra-korporalen Verlagerung der Energieversorgung entgegen. Weiterhin sind bei Patienten mit peripher arteriellem Verschluss (pAVK) und sogenannter Porzellanaorta (verkalkte Aorta) die bisherigen Systeme nur sehr eingeschränkt verwendbar.

### Kurzdarstellung der Erfindung

Die Erfindung hat sich zu einer Aufgabe gemacht, eine verbesserte Pumpe bereitzustellen, die einen oder mehrere Nachteile aus dem Stand der Technik löst.

Die Aufgabe wird gelöst durch eine Pumpe zur Implantierung in ein Gefäß oder ein Herz, wie beansprucht, wobei die Pumpe in einem ersten Zustand in das Gefäß oder Herz eingeführt wird, um anschließend im Gefäß oder im Herz funktional in einen zweiten Zustand überführt werden zu können. Die Pumpe weist einen Antriebsteil und einen Förderteil auf, wobei der Förderteil eine Antriebswelle aufweist, wobei der Antriebsteil in dem ersten Zustand nicht funktionsfähig und durch die Überführung in den zweiten Zustand funktionsfähig wird, wobei das Antriebsteil einen Elektromotor aufweist, wobei der Elektromotor als Nassläufer ausgebildet ist, und wobei in dem ersten Zustand der Rotor des Elektromotors und der Stator des Elektromotors räumlich getrennt voneinander angeordnet sind, sodass eine Rotation des Rotors (R) durch den Stator (S) nicht möglich ist, und wobei der Rotor des Elektromotors in dem zweiten Zustand in den Stator des Elektromotors verbracht ist, wobei der Rotor im zweiten Zustand das Förderteil antreiben kann.

### Kurzdarstellung der Figuren

Nachfolgend wird die Erfindung näher unter Bezugnahme auf die beigefügten Figuren erläutert werden. In diesen zeigt:
Fig. 1a eine beispielhafte schematische Darstellung in Bezug auf ein Prinzip der erfindungsgemäßen Pumpe in einem ersten Zustand in einer axialen Schnittdarstellung,
Fig. 1b eine beispielhafte schematische Darstellung in Bezug auf ein Prinzip der erfindungsgemäßen Pumpe in einem zweiten Zustand in einer axialen Schnittdarstellung,
Fig. 2 eine beispielhafte schematische Anordnung einer weiteren Ausführungsform einer erfindungsgemäßen Pumpe in einem zweiten Zustand im Myokard,
Fig. 3a eine beispielhafte schematische Anordnung einer weiteren Ausführungsform einer erfindungsgemäßen Pumpe in einem ersten Zustand in einer axialen Schnittdarstellung,
Fig. 3b eine beispielhafte schematische Anordnung einer weiteren Ausführungsform einer erfindungsgemäßen Pumpe gemäß Figur 3a in einem zweiten Zustand in einer axialen Schnittdarstellung,
Fig. 4a eine beispielhafte schematische Anordnung einer weiteren Ausführungsform einer erfindungsgemäßen Pumpe in einem ersten Zustand in einer axialen Schnittdarstellung,
Fig. 4b eine beispielhafte schematische Anordnung einer weiteren Ausführungsform einer erfindungsgemäßen Pumpe gemäß Figur 4a in einem zweiten Zustand in einer axialen Schnittdarstellung,
Fig. 5a eine beispielhafte schematische Anordnung in einer axialen Schnittdarstellung in Bezug auf einen Aspekt in einem ersten (geschlossenen) Zustand,
Fig. 5b eine beispielhafte schematische Anordnung in einer axialen Schnittdarstellung gemäß Figur 6a in Bezug auf einen Aspekt in einem zweiten (offenen) Zustand,
Fig. 6a eine beispielhafte schematische Anordnung in einer axialen Schnittdarstellung in Bezug auf einen Aspekt in einer weiteren Ausführungsform in einem ersten (geschlossenen) Zustand,
Fig. 6b eine beispielhafte schematische Anordnung in einer axialen Schnittdarstellung gemäß Figur 6a in Bezug auf einen Aspekt in einer weiteren Ausführungsform in einem zweiten (offenen) Zustand,
Fig. 7 eine beispielhafte Anordnung von Einheiten einer erfindungsgemäßen Pumpe in Bezug auf einen menschlichen Körper.

### Ausführliche Beschreibung

Gemäß der vorliegenden Erfindung wird eine Pumpe 1 zur Implantierung in ein Gefäß oder ein Herz vorgeschlagen. Obwohl diese in unterschiedlichen Gefäßsystemen zum Einsatz kommen kann, wird nachfolgend nur Bezug auf die Implantierung im Herzen, bzw. dem Herzmuskel, oder einem Herzgefäß Bezug genommen werden, ohne dass hierdurch die Allgemeinheit der Erfindung beschränkt ist.

Dabei erlaubt die Ausgestaltung der erfindungsgemäßen Pumpe 1 die Implantierung mittels sogenannter minimal-invasiver Technik. Beispielsweise kann die Pumpe 1 auf einen Führungsdraht montiert werden und mittels herkömmlicher Katheter-Technik an den Einsatzort vorgeschoben bzw. nur über einen Führungsdraht geschoben und dort befestigt und zum Einsatz gebracht werden. Andere Techniken der Einbringung an den Einsatzort sind hierdurch jedoch nicht ausgeschlossen, sondern sind für den Fachmann ohne weiteres ersichtlich. Beispielsweise erlaubt die erfindungsgemäße Pumpe 1 auch die Einbringung mittels Katheter oder einer Schleuse.

Hierfür wurde die Pumpe 1 erfindungsgemäß so gestaltet, dass sie zum einen die notwendige Pumpleistung erbringen kann und dennoch so klein gestaltet werden kann, dass sie durch ein entsprechendes Gefäß (Lumen) an den Einsatzort transportiert werden kann, ohne dieses Gefäß zu verletzen.

Durch die Gestaltung der Pumpe 1 wird es zudem ermöglicht, die Pumpe 1 apikal oder atrial in das Herz eines Patienten einzubringen.

Dies ist besonders bei verkalkten Gefäßen, z.B. bei einer Porzellanaorta, von großem Vorteil.

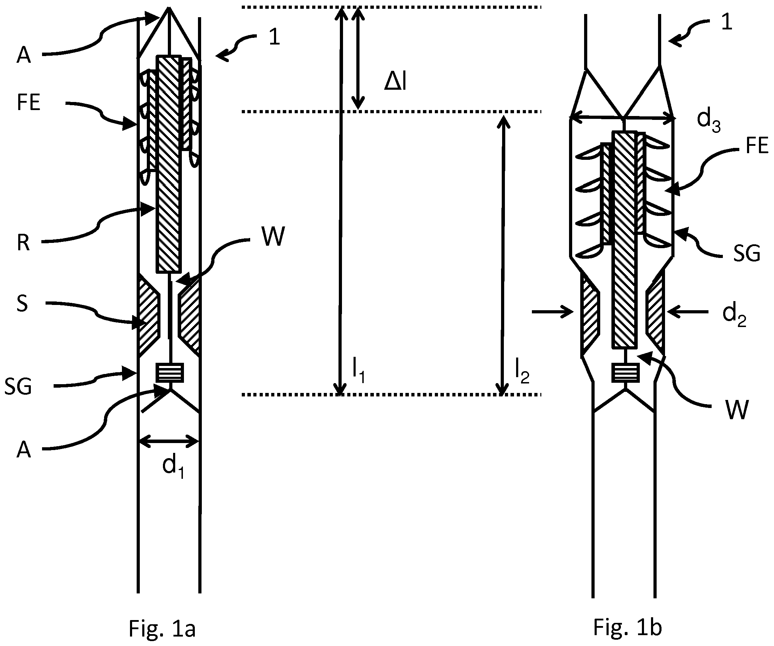

Ein Grundprinzip der Erfindung ist in den Fig. 1a und 1b. dargestellt.

Hierbei wird eine beispielhafte schematische Darstellung in Bezug auf ein Prinzip der erfindungsgemäßen Pumpe in einem ersten Zustand zur Verbringung an den Einsatzort (Fig.1a) und in einem zweiten Zustand betriebsfertig am Einsatzort jeweils in einer axialen Schnittdarstellung gezeigt.

Dabei wird eine Pumpe 1 zur Implantierung in ein Gefäß oder ein Herz zunächst an einer geeigneten Stelle in den Körper eingebracht. Dabei befindet sich die Pumpe 1 in einem ersten Zustand welcher in Figur 1a gezeigt ist, um anschließend im Gefäß oder im Herz funktional in einen zweiten Zustand überführt werden zu können.

Zum Transport an den Einsatzort wird die Pumpe 1 in ihrem ersten Zustand beispielsweise auf einen Führungsdraht eines geeigneten Katheters montiert bzw. über einen Führungsdraht geschoben. Die Pumpe 1 an sich weist einen Antriebsteil und einen Förderteil FE auf. Im ersten Zustand (Fig. 1a) ist der Antriebsteil noch nicht funktionsfähig. Insbesondere ist hier der Rotor (R) noch räumlich separiert vom Stator (S) angeordnet. In Fig. 1a beispielsweise ist der Rotor räumlich getrennt vor dem Stator angeordnet als auch der Stator noch in einem kollabierten Zustand dargestellt. Der Antriebsteil wird erst durch die Überführung in den zweiten Zustand funktionsfähig. In den Figuren 1a und 1b ist der Förderteil über eine Antriebswelle W mit dem Antriebsteil verbunden.

Das Antriebsteil weist dabei einen Elektromotor auf, wobei der Elektromotor als Nassläufer ausgebildet ist. Nassläufer sind besonders vorteilhaft, da diese einen vergleichsweise guten Durchfluss erlauben und zudem keine Probleme dahingehend aufweisen, dass der Motor besonders gekapselt werden muss, sodass zum einen der Motor nicht durch Blut oder seine Bestandteile verklebt oder verstopft oder korrodiert wird zum anderen aber auch keine Gefährdung für den Blutkreislauf durch Austausch von Schmierstoffen mit dem Blut entstehen kann.

Zum Transport ist in dem ersten Zustand der Rotor R des Elektromotors in aller Regel getrennt vom Stator S des Elektromotors angeordnet.

In der Figur 1a ist der Stator S und der Rotor R noch räumlich separiert, und daher ist der Motor nicht betriebsfähig. Erst durch die Einbringung der Welle wird der Stator S und der Rotor R ineinander verbracht, sodass der der Rotor R im Stator S drehbar wird und dann das Förderteil FE in Rotation versetzen kann (Fig. 1b).

Das Verbringen kann z.B. durch ein Verkürzen der Welle, ein Einschieben oder Verschieben bewerkstelligt werden. Besonders einfach ist es ein Verkürzen der Welle durch ziehen eines Drahtes (z.B. über einen Katheter) zu bewerkstelligen. Hierzu können unterschiedliche Techniken zum Einsatz kommen. Beispielhafte Techniken umfassen Zug oder Schub ausgeübt mittels eines Führungsdrahtes an einem Katheter oder mittels Formgedächtnislegierungen oder durch Federkräfte, etc. So kann z.B. die Pumpe im ersten Zustand federartig gespannt sein und bei Lösen einer Verriegelungseinrichtung oder bei Freisetzen einer in Form haltenden Ummantelung (z.B. Schleuse oder Katheter) wird die gespeicherte Kraft frei und führt zu einer Expansion bzw. zu einem Zusammensetzen der Einzelteile.

Im zweiten Zustand, der am Einsatzort gegeben ist, kann der Rotor R dann das Förderteil FE insbesondere über eine mechanische Verbindung, in der Regel eine Antriebswelle W, antreiben. Dieser Zustand ist beispielsweise in Figur 1b gezeigt.

Eine Verkürzung der Welle W ist beispielhaft in den Zeichnungen angeben. Insbesondere aus Fig. 1a und 1b kann ohne weiteres ersehen werden, dass die Länge l₁ im ersten Zustand in eine Länge l₂ überführt wird, wobei l₁>l₂ ist. D.h. die Welle wird um Δl beim Übergang in den zweiten Zustand verkürzt.

Dabei kann zudem der Durchmesser d₁ im Bereich des Stators S im zweiten Zustand je nach Ausgestaltung leicht in einen Durchmess d₂ ausgeweitet sein. Zudem kann der Bereich der Fördereinheit auf einen Durchmesser d₃ ausgeweitet sein. Obwohl in der Darstellung d₃>d₂ dargestellt ist, muss dies nicht notwendigerweise so sein, sondern ist nur zur Illustration gedacht, um aufzuzeigen, dass unterschiedliche Bereiche im betriebsfähigem zweiten Zustand unterschiedlich ausgeweitet sein können. In aller Regel ist der Durchmesser d₁ im ersten Zustand so gewählt, dass eine Applikation mit einem Katheter mit einem Durchmesser von 32 French oder weniger, insbesondere von 24 French oder weniger, möglich ist. Die Ausweitung der Durchmesser kann gleichzeitig oder hintereinander erfolgen.

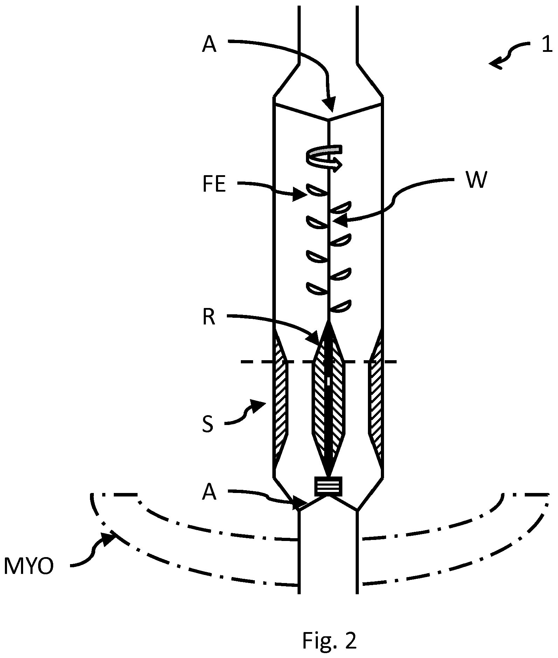

Wie bereits angedeutet, kann die fertige Pumpe 1 zum einen unmittelbar in oder an einem Gefäß bzw. im Herz verankert werden. So ist z.B. die Verankerung im Herzmuskel (lat. Myocardium) MYO innerhalb des Herzens wie in Figur 2 in Bezug auf eine mögliche Ausführungsform der Erfindung gezeigt. Ohne Beschränkung der Allgemeinheit kann natürlich auch ein anderer Einsatzort je nach Verwendung der erfindungsgemäßen Pumpe 1 vorgesehen sein.

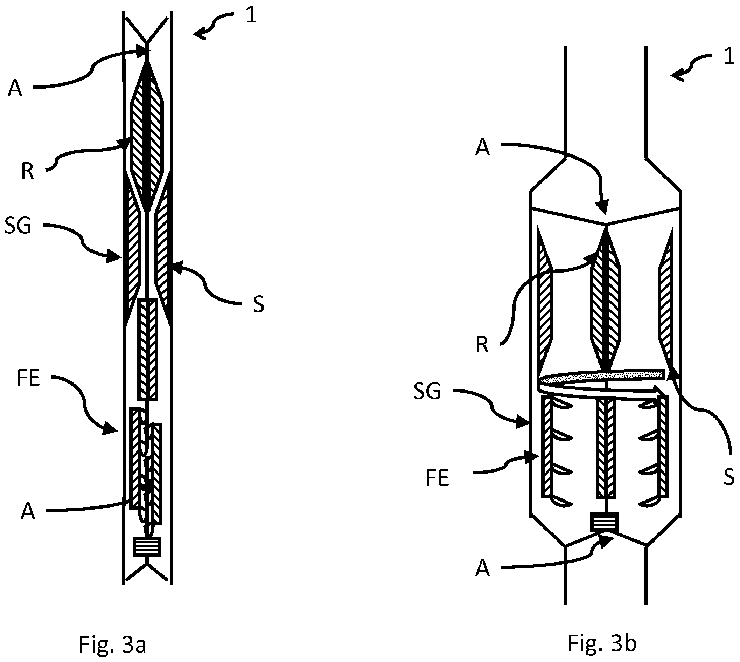

In Figur 3a und 3b ist eine weitere beispielhafte Gestaltung der Pumpe 1 in axialem Querschnitt in einem ersten Zustand, welcher in Figur 3a gezeigt ist, zur Verbringung an den Einsatzort und in einem zweiten Zustand, welche in Figur 3b gezeigt ist, betriebsfertig am Einsatzort gezeigt. In Bezug auf einen radialen Querschnitt ergäben sich ähnliche Ansichten, sodass hier auf eine entsprechende Wiedergabe verzichtet wird.

Dabei wird eine Pumpe 1 zur Implantierung in ein Gefäß oder ein Herz zunächst an einer geeigneten Stelle in das Herz-Kreislauf-System des Körpers eingebracht. Dabei befindet sich die Pumpe 1 in einem ersten Zustand welcher in Figur 3a gezeigt ist, um anschließend im Gefäß oder im Herz funktional in einen zweiten Zustand überführt werden zu können.

Zum Transport an den Einsatzort wird die Pumpe 1 in ihrem ersten Zustand beispielsweise auf einen Führungsdraht eines geeigneten Katheters montiert bzw. über einen Führungsdraht geschoben.

Alternativ kann der Transport auch über einen Katheter oder eine Schleuse ohne Führungsdraht erfolgen. Die Pumpe 1 an sich weist einen Antriebsteil und einen Förderteil FE auf. Im ersten Zustand (Fig. 3a) ist der Antriebsteil noch nicht funktionsfähig. Der Antriebsteil wird erst durch die Überführung in den zweiten Zustand funktionsfähig. Der Förderteil FE ist hier auf einem aufdehnbaren Segmenten angeordnet, die vom Rotor R (magnetisch gekoppelt) angetrieben werden. Hierzu weist die Pumpe 1 weiterhin eine Stützform SG auf, sodass das Förderteil FE nicht an der Wand des Gefäßes oder des Herzens reibt. Dabei können unterschiedliche Formen des Antriebes realisiert werden. So kann z.B. eine magnetische Lagerung und Kopplung als auch eine rein mechanische Lagerung oder eine magnetisch-mechanische vorgesehen werden.

Das Antriebsteil weist dabei einen Elektromotor auf, wobei der Elektromotor als Nassläufer ausgebildet ist. Nassläufer sind besonders vorteilhaft, da diese einen vergleichsweise guten Durchfluss erlauben und zudem keine Probleme dahingehend aufweisen, dass der Motor besonders gekapselt werden muss, sodass zum einen der Motor nicht durch Blut oder seine Bestandteile verklebt oder verstopft oder korrodiert wird zum anderen aber auch keine Gefährdung für den Blutkreislauf durch Austausch von Schmierstoffen mit dem Blut entstehen kann.

Zum Transport ist in dem ersten Zustand der Rotor R des Elektromotors in aller Regel getrennt vom Stator S des Elektromotors angeordnet.

In der Figuren 3a ist der Stator S und der Rotor R im ersten Zustand zunächst hintereinanderliegend, d.h. auf unterschiedlichen Ebenen, angeordnet und der Motor ist nicht betriebsfähig. Erst durch die Aufweitung im Bereich des Stators S wird der Rotor R drehbar und kann dann das Förderteil FE in Rotation versetzen (Fig. 3b).

Erst in dem zweiten Zustand am Einsatzort wird der Rotor R des Elektromotors in den Stator S des Elektromotors verbracht. Hierzu können unterschiedliche Techniken zum Einsatz kommen. Beispielhafte Techniken umfassen Zug oder Schub ausgeübt mittels eines Führungsdrahtes an einem Katheter oder mittels Formgedächtnislegierungen, oder durch Federkräfte, etc. So kann z.B. die Pumpe im ersten Zustand federartig gespannt sein und bei Lösen (einer Verriegelungseinrichtung) wird die gespeicherte Kraft frei und führt zu einer Expansion bzw. zu einem Zusammensetzen der Einzelteile. Dabei kann wie in der Figur 3b im Vergleich zu der Figur 3a gezeigt eine äußere Stützform SG entfaltet werden. So kann z.B. durch die Expansion der hüllenartigen Stützform SG auch dazu genutzt werden das Antriebsteil betriebsfähig zusammenzusetzen, z.B. durch eine sich verkürzende Welle. Diese Aufdehnung kann dabei neben der Erweiterung des Lumens, um eine Pumpleistung zur Verfügung zu stellen, auch die Funktion einer Abstützung der Pumpe 1 im Gefäß oder im Herzen übernehmen.

Im zweiten Zustand, der am Einsatzort gegeben ist, kann der Rotor R dann das Förderteil FE über die Antriebswelle W antreiben. Dieser Zustand ist in Figur 3b gezeigt.

In der Ausführungsform der Figur 3a und 3b ist das Förderteil FE magnetisch an das Antriebsteil gekoppelt. Hierdurch kann auch das Förderteil FE so gestaltet werden, dass es im ersten Zustand einen sehr geringen Querschnitt einnimmt. Dabei ist z.B. im ersten Zustand das Förderteile FE in zwei Abschnitten hintereinander liegend angeordnet. Im zweiten Zustand wird z.B. durch Selbstexpansion oder durch entsprechende andere Manipulationen der erste Abschnitt des Förderteils in den zweiten Abschnitt hingeschoben oder hineingezogen. Alternativ kann die Fördereinheit FE direkt über eine gemeinsame Welle mit der Antriebseinheit verbunden sein.

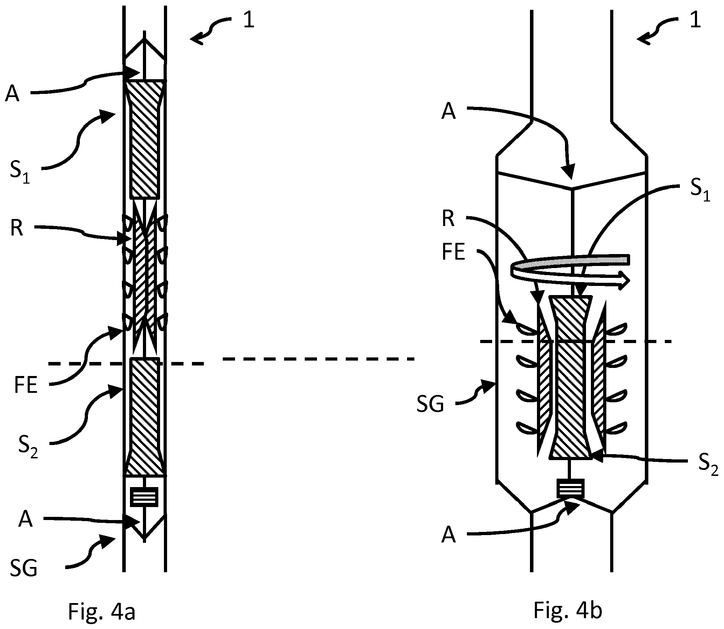

In Figur 4a und 4b ist hierzu noch eine weitere beispielhafte Gestaltung in axialem Querschnitt in einem ersten Zustand, welcher in Figur 4a gezeigt ist, zur Verbringung an den Einsatzort und in einem zweiten Zustand, welcher in Figur 4b gezeigt ist, betriebsfertig am Einsatzort gezeigt. In Bezug auf einen radialen Querschnitt ergäben sich ähnliche Ansichten, sodass hier auf eine entsprechende Wiedergabe verzichtet wird.

Dabei wird eine Pumpe 1 zur Implantierung in ein Gefäß oder ein Herz zunächst an einer geeigneten Stelle in das Herz-Kreislauf-System des Körpers eingebracht. Dabei befindet sich die Pumpe 1 in einem ersten Zustand, welcher in Figur 4a gezeigt ist, um anschließend im Gefäß oder im Herz funktional in einen zweiten Zustand überführt werden zu können.

Zum Transport an den Einsatzort wird die Pumpe 1 in ihrem ersten Zustand beispielsweise auf einen Führungsdraht eines geeigneten Katheters montiert bzw. über einen Führungsdraht geschoben. Alternativ kann der Transport auch über eine Katheter oder eine Schleuse ohne Führungsdraht erfolgen. Die Pumpe 1 an sich weist einen Antriebsteil und einen Förderteil FE auf. Im ersten Zustand (Fig. 4a) ist der Antriebsteil noch nicht funktionsfähig. Der Antriebsteil wird erst durch die Überführung in den zweiten Zustand funktionsfähig. In der Ausführungsform der Figuren 4a und 4b ist der Förderteil FE beispielsweise auf dem Rotor R angebracht.

Das Antriebsteil weist dabei einen Elektromotor auf, wobei der Elektromotor beispielsweise als Nassläufer ausgebildet ist. Nassläufer sind besonders vorteilhaft, da diese einen vergleichsweise guten Durchfluss erlauben und zudem keine Probleme dahingehend aufweisen, dass der Motor besonders gekapselt werden muss, sodass zum einen der Motor nicht durch Blut oder seine Bestandteile verklebt oder verstopft oder korrodiert wird zum anderen aber auch keine Gefährdung für den Blutkreislauf durch Austausch von Schmierstoffen mit dem Blut entstehen kann.

Zum Transport ist in dem ersten Zustand der Rotor R des Elektromotors in aller Regel getrennt vom Stator S des Elektromotors angeordnet.

In der Figur 4a ist der Stator S beispielsweise in zwei Teile S₁ und S₂ geteilt und der Rotor R im ersten Zustand zunächst dazwischen liegend, d.h. auf unterschiedlichen Ebenen, angeordnet und der Motor ist nicht betriebsfähig. Erst durch die Positionierung des Stators S im Rotor R wird der Rotor R drehbar und kann dann das Förderteil FE in Rotation versetzen (Fig. 4b). Dabei werden die beiden Statorteile S₁ und S₂ räumlich angenähert. Alternativ kann beim Wechsel von dem ersten Zustand in den zweiten Zustand auch nur von einer Seite der Stator S (oder ein Teil davon S₁, S₂) in den Rotor R eingebracht werden.

Erst beim Übergang in den zweiten Zustand am Einsatzort werden Rotor R und Stator S des Elektromotors ineinander verbracht. Hierzu können unterschiedliche Techniken zum Einsatz kommen. Beispielhafte Techniken umfassen Zug oder Schub mittels eines Führungsdrahtes an einem Katheter, Formgedächtnislegierungen, Federkräfte, etc. So kann z.B. die Pumpe im ersten Zustand federartig gespannt sein und bei Lösen (einer Verriegelungseinrichtung) wird die gespeicherte Kraft frei und führt zu einer Expansion bzw. zu einem Zusammensetzen der Einzelteile. Dabei kann wie in der Figur 1b im Vergleich zu der Figur 1a gezeigt eine äußere Stützform SG entfaltet und eventuell das entsprechende Gefäß oder Myokard MYO aufgedehnt werden. Diese Aufdehnung kann dabei neben der Erweiterung des Lumens, um eine Pumpleistung zur Verfügung zu stellen, auch die Funktion einer Abstützung der Pumpe 1 im Gefäß oder Myokard MYO übernehmen.

Im zweiten Zustand, der am Einsatzort gegeben ist, kann der Rotor R dann das Förderteil FE antreiben. Dieser Zustand ist in Figur 4b gezeigt.

Um vom ersten in den zweiten Zustand zu gelangen, kann beispielsweise eine sich verkürzende Welle vorgesehen sein. Beispielsweise kann diese Änderung sich dadurch ergeben, dass die Befestigungseinrichtung A in Fig. 4a und 4b relativ zur Welle als auch relativ zur Stützform SG die Ausrichtung ändert.

In einer besonders einfachen Ausgestaltung ist zudem vorgesehen, dass die Antriebswelle W und der Rotor R einstückig ausgeführt sind. Hierdurch können Produktionskosten eingespart werden und Fehlerquellen minimiert werden. Auf der anderen Seite können der Rotor R und die Antriebswelle W beispielsweise auch mehrstückig ausgebildet sein, sodass z.B. eine höhere Flexibilität beim Transport gegeben ist. Hierdurch wird z.B. die Einbringung durch bereits stark verkalkte Gefäße deutlich vereinfacht, da so flexiblere Pumpen 1 transportiert werden können, die erst bei Einsatz zusammengesetzt werden.

In einer alternativen Ausführung kann auch der Rotor im ersten Zustand in zwei oder mehr Teile zerlegt sein, die wiederum erst bei Überführung in den zweiten Zustand ineinander gefügt werden.

Ohne Beschränkung der Allgemeinheit erlaubt die Erfindung, die Verwendung unterschiedlicher Lager. Beispielsweise können mechanische Lagerungen zum Einsatz kommen. Dabei werden besonders vorteilhaft abriebsbeständige Materialien für das Lager an sich oder aber Beschichtungen einzelner Teile der Lager verwendet. Beispielhafte Materialien weisen Keramik oder edle Stähle auf. Insbesondere wird jedoch eine magnetische Lagerung oder eine magneto-mechanische Lagerung oder eine magneto-hydromechanische Lagerung der Antriebswelle W als auch des Rotors R unterstützt. Insbesondere magnetische Lagerungen erweisen sich als vorteilhaft, da bei diesen die Reibung minimiert werden kann und deren Verwendung in Zusammenhang mit Nassläufern besonders vorteilhaft ist. Insbesondere erlaubt die magnetische Lagerung (als auch die magnetisch unterstützte magneto-mechanische bzw. magneto-hydromechanische Lagerung), dass der für den Durchfluss zur Verfügung stehende Querschnitt vergrößert wird als auch Verschleiß minimiert wird. Dies wirkt sich vorteilhaft auf die Förderleistung und/oder auf den Energieverbrauch aus. Beispielsweise kann mit der Anordnung aus Figur 4a und 4b besonders einfach eine axiale magnetische Lagerung bereitgestellt werden, während mit den Anordnungen aus den Figuren 2 und 3 besonders einfach radiale magnetische Lagerungen hergestellt werden können. Da die Figuren nur Prinzipien darstellen, können auch Mischformen aus radialen und axialen Lagerungen realisiert werden.

Um die Einbringung der erfindungsgemäßen Pumpe 1 und die Verbringung von dem ersten Zustand in den zweiten Zustand zu ermöglichen, kann z.B. vorgesehen sein, dass die Zustandsänderung durch einen Wärmeeintrag und/oder eine ausgeübte mechanische Kraft und/oder ein äußeres Magnetfeld und/oder ein inneres Magnetfeld hervorgerufen wird. Ein Wärmeeintrag kann z.B. durch die äußere Einkoppelung eines Wirbelstromes, eine intern eingebrachte Wärmequelle und/oder eine Kombination von beidem hervorgerufen werden. Dabei kann zum einen direkte Erwärmung von (Teilen der) Pumpe 1 oder eine indirekte Wärmeübertragung auf (Teile der) Pumpe 1 vorgesehen sein. Dabei ist es von Vorteil nur die Teile zu Erwärmen, die auch eine wärmeinduzierte Zustandsänderung zulassen. Es besteht darüber hinaus die Möglichkeit, dass der Wärmeeintrag durch die Körpertemperatur erfolgen kann. Hierzu ist gegebenenfalls vor Implantation eine Kühlung von (zumindest Teilen der) Pumpe 1 erforderlich. Alternativ oder Zusätzlich kann eine mechanische Kraft mittels eines Führungsdrahtes oder einem oder mehrerer weiterer Manipulationsdrähte, wie sie in Katheter Verwendung finden, benutzt werden.

Insbesondere ist es vorteilhaft, wenn das Förderteil FE und/oder der Rotor R und/oder der Stator S und/oder ein äußere Stützform SG eine Formgedächtnislegierung bzw. eine Selbstexpansionsfähigkeit aufweisen. Diese Materialien erlauben eine besonders einfach herstellbare Struktur, die sich beispielsweise durch Erwärmung oder Abkühlung, von dem ersten Zustand in einen anderen Zustand verbringen lässt. Zudem sind diese Materialen bereits als Gefäßstützen erprobt und weisen eine gute Biokompatibilität auf.

In vorteilhaften Ausführungsformen weist die Pumpe 1 eine Fixiereinrichtung zur Fixierung im Herz (z.B. Herzmuskel Myokard) und/oder dem Gefäß auf. Beispielhafte Ausführungsformen können ankerartig oder angelhakenartig oder nietenkopf ausgestalt sein. Hiermit wird eine sichere Verankerung im Gefäß oder im Herzen bereitgestellt. Alternativ kann die Pumpe 1 auch über eine ringförmige Struktur am Myokard z.B. durch entsprechendes (chirurgisches) Nahtmaterial oder Klebemittel befestigt werden. Ohne Beschränkung der Allgemeinheit können dabei auch mehrere unterschiedliche Fixiereinrichtungen vorgesehen sein.

Um eine weitere Reduktion des Querschnittes im ersten Zustand bereitzustellen, kann zudem vorgesehen sein, dass auch der Stator S in einem ersten Zustand komprimiert ist und durch die Überführung in den zweiten Zustand expandiert wird.

Ebenfalls um eine weitere Reduktion des Querschnittes im ersten Zustand bereitzustellen, kann zudem vorgesehen sein, dass das Förderteil FE in einem ersten Zustand nicht funktionsfähig ist und durch die Überführung in den zweiten Zustand funktionsfähig wird. Beispielsweise kann auch die Fördereinrichtung FE oder Teile hiervon eine Formgedächtnislegierung aufweisen, das erst mit Aktivierung (oder auch nach Aktivierung) des zweiten Zustandes zu einem Entfalten der Fördereinrichtung führt.

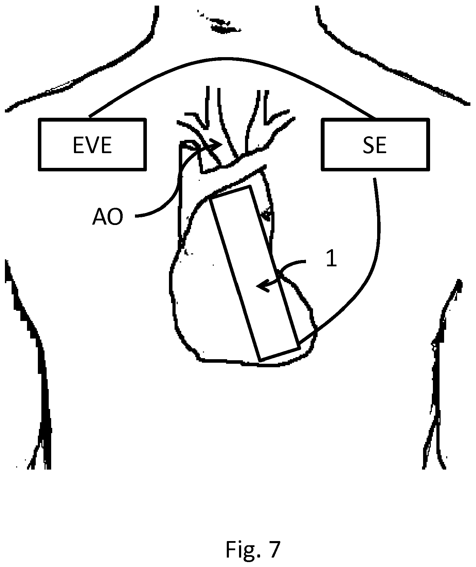

Wie in Figur 7 gezeigt kann der Pumpe 1 eine Energieversorgungseinheit EVE zugeordnet sein.

In vorteilhaften Ausführungsformen wird die Energieversorgungseinheit EVE durch Induktion geladen. Hierdurch werden Probleme mit Infektionen durch Anschlussdrähte, welche in das Körperinnere führen vermieden und es wird eine intra-korporale Anordnung ermöglicht werden.

Eine induktive Aufladung trägt zum Patientenkomfort bei, da diese nun ohne großen Aufwand, z.B. in Ruhephasen, die Energieversorgungseinheit aufladen können, ohne durch Kabel gestört zu sein.

Weiterhin ist eine intra-korporale Energieversorgungseinheit EVE insofern vorteilhaft, weil der damit ausgestattete Patient einen größeren Freiraum genießt und nicht kontinuierlich auf die Verbindungskabel Rücksicht nehmen muss. Dennoch kann vorgesehen sein, ohne Einschränkung der Allgemeinheit dass die Energieversorgungseinheit EVE auch extra-korporal angeordnet ist. Dies kann z.B. dann vorteilhaft sein, wenn die Energieversorgungseinheit EVE zu groß im Vergleich zum verfügbaren Raum wäre. Dies kann z.B. dann der Fall sein, wenn die Pumpe 1 bei Kindern und Jugendlichen implantiert wird oder aber der Energiebedarf so hoch ist, dass die notwendige Akkuleistung für eine sinnvollen Betrieb zu einer zu großen und/oder zu schwere Energieversorgungseinheit EVE führen würde. Ohne Beschränkung der Allgemeinheit, kann die Energieversorgungseinheit EVE auch in mehreren (auch verteilt angeordneten) Teileinheiten verkörpert sein.

Besonders vorteilhaft ist diese Energieversorgungseinheit EVE ebenso wie die Pumpe 1 minimalinvasiv implantierbar.

In Ausführungsformen der Erfindung kann zudem der Pumpe 1 eine Steuereinheit SE zugeordnet sein, welche anhängig von einem zu fördernden Volumen, einen vorhandenen Volumenstrom unterstützt.

Ohne Beschränkung der Allgemeinheit kann die Steuereinheit SE extra-korporal oder intra-korporal angeordnet ist. Besonders vorteilhaft ist jedoch eine intra-korporale Steuereinheit SE, da diese die Gefahr von Infektionen minimiert. Weiterhin ist eine intra-korporale Steuereinheit SE insofern vorteilhaft, weil der damit ausgestattete Patient einen größeren Freiraum genießt und nicht ständig auf die Verbindungskabel Rücksicht nehmen muss.

Alternative oder zusätzlich kann zudem vorgesehen sein, dass die Pumpe 1 manuell und/oder automatisch gesteuert werden kann. Durch eine manuelle Steuerung kann z.B. gezielt durch den Patienten sobald er merkt oder weiß, dass er einen erhöhten Pumpbedarf hat oder haben wird, die Pumpleistung entsprechend angepasst werden. Auch kann z.B. durch entsprechendes medizinisches Personal hier Einfluss genommen werden. Die manuelle Steuerung kann dabei in vielerlei Gestalt realisiert sein. Beispielsweise kann eine mechanische Steuerung mittels (Druck-) Schaltern, welche beispielsweise unter der Haut tast- und schaltbar angeordnet sind, bereitgestellt erfolgen. Alternativ oder zusätzlich kann eine Funksteuerung oder eine andere elektromagnetisch gekoppelte Steuerung vorgesehen sein.

Die automatische Steuerung kann dabei unterschiedlich ausgestaltet sein. Beispielsweise kann eine zeitgesteuerte Steuerung den Tagesablauf berücksichtigen, sodass z.B. in Ruheperioden eine geringere oder gar keine Unterstützung zur Verfügung gestellt wird. In anderen Beispielen kann die automatische Steuerung auch eine hämodynamische Steuerung aufweisen. Beispielsweise kann durch eine geeignete Einrichtung der Blutfluss erkannt werden, und wenn ein hoher Blutfluss abgerufen wird, eine entsprechende Unterstützung bereitstellen. Geeignete Parameter zur Erkennung des Blutflusses können z.B. aus dem Herzzeitvolumen, dem Puls und/oder dem Blutdruck abgeleitet werden. Dies kann z.B. Pulsgesteuert oder alternativ oder zusätzlich Blutdruckgesteuert oder aber alternativ oder zusätzlich über die Pumpe 1 selbst, welche z.B. im ausgeschalteten Zustand als Durchflussmesser fungiert, bereitgestellt werden. Weiterhin kann die automatische Steuerung auch mittels einer Bewegungssteuerung realisiert werden. Wird ein gewisses Maß an Bewegung mittels Bewegungssensoren, wie sie z.B. aus Smartphones, Navigationsgeräten oder dergleichen bekannt sind, erkannt, so wird eine entsprechende Unterstützung aktiviert. Ohne weiteres können auch Mischformen der oben aufgezeigten automatischen Steuerungen zum Einsatz kommen.

Somit wird es ermöglicht die Pumpe 1 nur dann zu aktivieren, wenn ein entsprechend erhöhtes Herzzeitvolumen benötigt wird. Diese Aktivierbarkeit nach Notwendigkeit erlaubt es den Energieverbrauch zu minimieren und erlaubt so eine vorteilhafte Ausgestaltung mit intra-korporaler Steuereinheit SE und/oder intra-korporaler Energieversorgungseinheit EVE, welche eine deutliche Verbesserung der Lebensqualität der Patienten darstellt.

Ohne weiteres können die Steuereinheit SE und die Energieversorgungseinheit EVE auch als eine integrierte Einheit ausgeführt sein, d.h. soweit vorstehend die Einheiten getrennt dargestellt wurden, ist diese Trennung nicht notwendigerweise physikalisch zu verstehen sondern dient lediglich zur Illustration der unterschiedlichen Aufgaben.

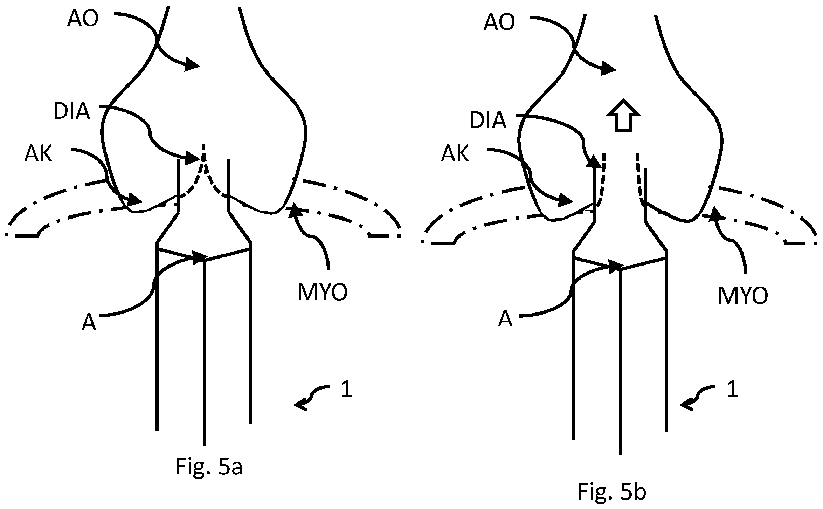

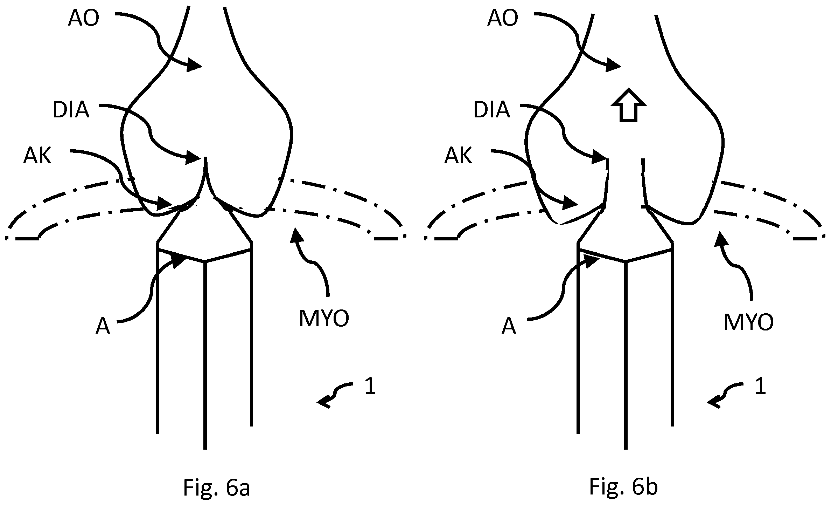

Durch eine entsprechende Gestaltung wird zudem ein retrograder Fluss verhindert. Dies wird z.B. durch ein Diaphragma DIA, wie in Fig. 5a und 5b bzw. 6a und 6b gezeigt verhindert. Dabei stellen diese gezeigten Ausführungsvarianten keine Limitierung dar und andere Ausführungsformen, um einen Rückfluss zu verhindern, wie entsprechend geformte Rückschlagventile, sind ebenso umfasst. In Fig. 5a und 5b ist beispielhaft für eine Einrichtung zur Verhinderung eines Rückflusses ein als gestrichelte Linie dargestelltes Diaphragma in die Pumpe 1 integriert bzw. angebaut, wobei hier der Ausflusstrakt der Pumpe 1 über der Aortenklappe bis in die Aorta ascendens AO angeordnet ist. Alternativ kann an dieser Stelle auch eine biologische oder mechanische Klappe eingebracht sein, die den Rückfluss einschränkt oder unterbindet. In Fig. 5a und 5b ist das Pumpenende Diaphragma-artig ausgeführt. Dabei stellen die Figuren 5a und 6a jeweils den Fall dar, in dem ein retrograder Fluss durch die Pumpe 1 verhindert wird, wohingegen die Figuren 5b und 6b jeweils einen Fall darstellen, in dem ein antegrader Durchfluss aus der Pumpe ermöglicht wird.

Durch die generelle Positionierung der Pumpe 1 im Blutstrom wird zudem eine Thrombenbildung an der Pumpe 1 selbst im ausgeschalteten Zustand verhindert oder verringert.

Auch das Förderteil FE kann unterschiedlich ausgestaltet sein. In aller Regel wird jedoch das Förderteil FE zumindest abschnittsweise eine Schraubenform oder eine Propellerform aufweisen. Ohne Beschränkung der Allgemeinheit kann das Förderteil FE auch magnetisch mit dem Rotor R bzw. der Antriebswelle W gekoppelt sein.

Die vorliegende Erfindung erlaubt durch ihre Gestaltung die Einbringung mittels minimalinvasiver Techniken. Durch die Bereitstellung einer am Einsatzort in Betrieb zu setzenden Antriebsteil wird diese soweit transportfähig in ihrem radialen Querschnitt verkleinert, dass mittels der minimalinvasiver Techniken an den Einsatzort verbracht werden kann. So kann z.B. ein apikaler, ein atrialer oder eine transvaskulärer Zugang Verwendung finden.

Ohne Beschränkung der Allgemeinheit können auch weitere Bestandteile der Pumpe 1 Gegenstand von expandierbaren Mitteln sein. Beispielsweise können auch das Förderteil als auch weitere Bestandteile erst am Einsatzort betriebsfähig werden, z.B. dadurch, dass sie beim expandieren des Antriebsteils oder getrennt hiervon expandiert werden. Hierzu können für unterschiedliche Elemente der erfindungsgemäßen Pumpe 1 unterschiedliche Techniken oder aber mehrere Techniken gleichzeitig oder in bestimmter zeitlicher Folge verwendet werden. Beispielhafte Techniken umfassen Zug oder Schub mittels eines Führungsdrahtes an einem Katheter, Formgedächtnislegierungen, Federkräfte, etc.

Soweit vorstehend Bezug auf bestimmte Organe, wie z.B. das Herz oder den Herzmuskel (Myokard) genommen wurde, ist dies lediglich beispielhaft. Im Prinzip ist die Vorrichtung für jegliches Lumen im menschlichen oder tierischen Körper geeignet.

Aufgrund der Erfindung ist es insbesondere möglich erfindungsgemäße Pumpen bereitzustellen, die mittels eines 32 French (oder kleiner) Katheters, insbesondere eines 24 French oder kleiner Katheters in den Körper eingeführt werden können. Somit kann ohne weiteres eine minimal-invasive Applikation erreicht werden, wodurch auch Patienten behandelt werden können, die ansonsten auf Grund des hohen Risikos einer Operation nicht behandelt werden konnten. Zudem ist es nunmehr u.a. auch möglich Patienten zu behandeln, bei denen aufgrund der Größen bisheriger Pumpen oder Zuführungssysteme eine Verwendung von Pumpen nicht oder nur eingeschränkt möglich war (z.B. in der Pädiatrie, Neonatologie oder bei multimorbiden Patienten).

## Patentansprüche

1. Pumpe (1) zur Implantierung in ein Gefäß oder ein Herz, wobei die Pumpe (1) in einem ersten Zustand in das Gefäß oder das Herz eingeführt werden kann, um anschließend im Gefäß oder im Herz funktional in einen zweiten Zustand überführt werden zu können, aufweisend
• einen Antriebsteil und
• einen Förderteil (FE),
• wobei der Antriebsteil in dem ersten Zustand nicht funktionsfähig und durch die Überführung in den zweiten Zustand funktionsfähig wird,
• wobei das Antriebsteil einen Elektromotor aufweist,
• wobei der Elektromotor als Nassläufer ausgebildet ist, und
• wobei in dem ersten Zustand der Rotor (R) des Elektromotors und der Stator (S) des Elektromotors räumlich getrennt voneinander angeordnet sind, sodass eine Rotation des Rotors (R) durch den Stator (S) nicht möglich ist, und wobei der Rotor (R) des Elektromotors in dem zweiten Zustand in den Stator (S) des Elektromotors verbracht ist, wobei der Rotor (R) im zweiten Zustand das Förderteil (FE) antreiben kann,
• wobei der Förderteil eine Antriebswelle (W) aufweist.

2. Pumpe (1) nach Anspruch 1, wobei die Antriebswelle (W) und der Rotor (R) einstückig ausgeführt sind.

3. Pumpe (1) nach Anspruch 2, **dadurch gekennzeichnet, dass** die Antriebswelle (W) als sich beim Übergang vom ersten Zustand in den zweiten Zustand sich verkürzende Welle ausgelegt ist, die das Verbringen des Rotors (R) in den Stator (S) ermöglicht.

4. Pumpe (1) nach Anspruch 2 oder 3, **dadurch gekennzeichnet, dass** die Antriebswelle (W) magnetisch oder mechanisch oder magneto-mechanisch oder magneto-hydromechanische gelagert ist.

5. Pumpe (1) nach einem der vorhergehenden Ansprüche, **dadurch gekennzeichnet, dass** der Rotor (R) magnetisch oder mechanisch oder magneto-mechanisch oder magneto-hydromechanische gelagert ist.

6. Pumpe (1) nach einem der vorhergehenden Ansprüche, **dadurch gekennzeichnet, dass** die Zustandsänderung durch einen Wärmeeintrag und/oder eine ausgeübte mechanische Kraft und/oder ein äußeres Magnetfeld und/oder ein inneres Magnetfeld und/oder eine Selbstexpansion hervorgerufen wird.

7. Pumpe (1) nach einem der vorhergehenden Ansprüche, **dadurch gekennzeichnet, dass** das Förderteil (FE) und/oder der Rotor (R) und/oder der Stator (S) und/oder ein äußere Stützform (SG) ein Formgedächtnislegierung aufweisen.

8. Pumpe (1) nach einem der vorhergehenden Ansprüche, **dadurch gekennzeichnet, dass** die Pumpe (1) eine Fixiereinrichtung zur Fixierung im Herz und/oder dem Gefäß aufweist.

9. Pumpe (1) nach einem der vorhergehenden Ansprüche, **dadurch gekennzeichnet, dass** der Stator (S) in dem ersten Zustand komprimiert ist und durch die Überführung in den zweiten Zustand expandiert wird.

10. Pumpe (1) nach einem der vorhergehenden Ansprüche, **dadurch gekennzeichnet, dass** das Förderteil (FE) in dem ersten Zustand nicht funktionsfähig und durch die Überführung in den zweiten Zustand funktionsfähig wird.

11. Pumpe (1) nach einem der vorhergehenden Ansprüche, **dadurch gekennzeichnet, dass** der Pumpe (1) eine Energieversorgungseinheit (EVE) zugeordnet ist.

12. Pumpe (1) nach Anspruch 11, **dadurch gekennzeichnet, dass** die Energieversorgungseinheit (EVE) durch Induktion geladen werden kann.

13. Pumpe (1) nach Anspruch 11 oder 12, **dadurch gekennzeichnet, dass** die Energieversorgungseinheit (EVE) extra-korporal oder intra-korporal angeordnet sein kann.

14. Pumpe (1) nach einem der vorhergehenden Ansprüche, **dadurch gekennzeichnet, dass** der Pumpe (1) eine Steuereinheit (SE) zugeordnet ist, welche anhängig von einem zu fördernden Volumen, einen vorhandenen Volumenstrom unterstützt.

15. Pumpe (1) nach Anspruch 14, **dadurch gekennzeichnet, dass** die Steuereinheit (SE) extra-korporal oder intra-korporal angeordnet sein kann.

16. Pumpe (1) nach Anspruch 14 oder 15, **dadurch gekennzeichnet, dass** die Pumpe (1) manuell und/oder automatisch gesteuert werden kann.

17. Pumpe (1) nach Anspruch 16, wobei im Falle der automatischen Steuerung, die Steuerung ausgewählt ist aus einer Gruppe aufweisend zeitgesteuert, hämodynamische Steuerung, Bewegungssteuerung.

18. Pumpe (1) nach einem der vorhergehenden Ansprüche, **dadurch gekennzeichnet, dass** das Förderteil (FE) zumindest abschnittsweise eine Schraubenform oder eine Propellerform aufweist.

19. Pumpe (1) nach einem der vorhergehenden Ansprüche, **dadurch gekennzeichnet, dass** zumindest ein Teil der Pumpe (1) eine Beschichtung zur Reduktion von Reibung, Einwachsen, Ablagerungen aufweist.

20. Pumpe (1) nach einem der vorhergehenden Ansprüche, **dadurch gekennzeichnet, dass** die Pumpe (1) eine Einrichtung zur Verhinderung eines Rückflusses (DIA) aufweist.

21. Pumpe nach einem der vorhergehenden Ansprüche, **dadurch gekennzeichnet, dass** die Pumpe (1) einen Durchmesser aufweist, sodass sie durch einen Katheter mit Durchmesser French 32 oder weniger geführt werden kann.

## Claims

1. Pump (1) for implantation into a vessel or a heart, whereby the pump (1) may be introduced in a first state into the vessel or heart in order then be functionally changed over to a second state in the vessel or in the heart, comprising
• a drive part and
• a delivery part (FE),
• wherein the drive part is not functional in the first state and becomes functional as a result of the changeover to the second state,
• wherein the drive part has an electric motor,
• wherein the electric motor is embodied as a wet running rotor, and
• wherein, in the first state, the rotor (R) of the electric motor and the stator (S) of the electric motor are arranged so as to be spatially separate from one another, so that a rotation of the rotor (R) by the stator (S) is not possible, and wherein the rotor of the electric motor is moved into the stator (S) of the electric motor in the second state, wherein the rotor (R) can drive the delivery part (FE) in the second state,
• wherein the delivery part has a drive shaft (W).

2. The pump (1) as set forth in claim 1, wherein the drive shaft (W) and the rotor (R) are integrally formed.

3. The pump (1) as set forth in claim 2, **characterized in that** the drive shaft (W) is designed as a shaft that shortens upon the changeover from the first state to the second state, enabling the rotor (R) to be introduced into the stator (S).

4. The pump (1) as set forth in claim 2 or 3, **characterized in that** the drive shaft (W) is supported magnetically or mechanically or magnetomechanically or magneto-hydromechanically.

5. The pump (1) as set forth in any one of the preceding claims, **characterized in that** the rotor (R) is supported magnetically or mechanically or magneto-mechanically or magneto-hydromechanically.

6. The pump (1) as set forth in any one of the preceding claims, **characterized in that** the state change is brought about by a heat input and/or an applied mechanical force and/or an external magnetic field and/or an internal magnetic field and/or self-expansion.

7. The pump (1) as set forth in any one of the preceding claims, **characterized in that** the delivery part (FE) and/or the rotor (R) and/or the stator (S) and/or an external supporting structure (SG) has a shape memory alloy.

8. The pump (1) as set forth in any one of the preceding claims, **characterized in that** the pump (1) has a fixing means for fixation in the heart and/or the vessel.

9. The pump (1) as set forth in any one of the preceding claims, **characterized in that** the stator (S) is compressed in the first state and expanded as a result of the changeover to the second state.

10. The pump (1) as set forth in any one of the preceding claims, **characterized in that** the delivery part (FE) is not functional in the first state and becomes functional as a result of the changeover to the second state.

11. The pump (1) as set forth in any one of the preceding claims, **characterized in that** a power supply unit (EVE) is associated with the pump (1).

12. The pump (1) as set forth in claim 11, **characterized in that** the power supply unit (EVE) may be charged through induction.

13. The pump (1) as set forth in claim 11 or 12, **characterized in that** the power supply unit (EVE) is arranged extracorporeally or intracorporeally.

14. The pump (1) as set forth in any one of the preceding claims, **characterized in that** a control unit (SE) is associated with the pump (1) that supports an existing volume flow depending on a volume to be delivered.

15. The pump (1) as set forth in claim 14, **characterized in that** the control unit (SE) may be arranged extracorporeally or intracorporeally.

16. The pump (1) as set forth in claim 14 or 15, **characterized in that** the pump (1) can be controlled manual and/or automatically.

17. The pump (1) as set forth in claim 16, wherein, in the case of automatic control, the control system is selected from a group including time-controlled, hemodynamic control, and motion control.

18. The pump (1) as set forth in any one of the preceding claims, **characterized in that** the delivery part (FE) has a screw shape or a propeller shape at least in portions.

19. The pump (1) as set forth in any one of the preceding claims, **characterized in that** at least a portion of the pump (1) has a coating for reducing friction, adhesion, and deposits.

20. The pump (1) as set forth in any one of the preceding claims, **characterized in that** the pump (1) has a device (DIA) for preventing backflow.

21. The pump as set forth in any one of the preceding claims, **characterized in that** the pump (1) has a diameter which is such that it can be passed through a catheter having a diameter of French 32 or less.

## Revendications

1. Pompe (1), destinée à être implantée dans un vaisseau ou un cœur, dans un premier état, la pompe (1) pouvant être introduite dans le vaisseau ou dans le cœur, pour pouvoir être transférée fonctionnellement par la suite dans un second état, à l'intérieur du vaisseau ou du cœur, comportant
• une pièce d'entraînement et
• une pièce de refoulement (FE),
• la pièce d'entraînement n'étant pas opérationnelle dans le premier état et devenant opérationnelle par le transfert dans le second état,
• la pièce d'entraînement comportant un moteur électrique,
• le moteur électrique étant conçu sous la forme d'un rotor noyé, et
• dans le premier état, le rotor (R) du moteur électrique et le stator (S) du moteur électrique étant placés en étant physiquement séparés l'un de l'autre, de sorte qu'une rotation du rotor (R) par le stator (S) ne soit pas possible, et dans le second état, le rotor (R) du moteur électrique étant amené dans le stator (S) du moteur électrique, dans le second état, le rotor (R) pouvant entraîner la pièce de refoulement (FE),
• la pièce de refoulement comportant un arbre d'entraînement (W).

2. Pompe (1) selon la revendication 1, l'arbre d'entraînement (W) et le rotor (R) étant conçus en monobloc.

3. Pompe (1) selon la revendication 2, **caractérisée en ce que** l'arbre d'entraînement (W) est conçu sous la forme d'un arbre qui raccourcit lors du transfert du premier état dans le second état, qui permet d'amener le rotor (R) dans le stator (S).

4. Pompe (1) selon la revendication 2 ou 3, **caractérisée en ce que** l'arbre d'entraînement (W) est logé par voie magnétique ou mécanique ou magnéto-mécanique ou magnéto-hydromécanique.

5. Pompe (1) selon l'une quelconque des revendications précédentes, **caractérisée en ce que** le rotor (R) est logé par voie magnétique ou mécanique ou magnéto-mécanique ou magnéto-hydromécanique.

6. Pompe (1) selon l'une quelconque des revendications précédentes, **caractérisée en ce que** le changement d'état est provoqué par un apport de chaleur et/ou par l'exercice d'une force mécanique et/ou par un champ magnétique extérieur et/ou par un champ magnétique intérieur et/ou par une auto-expansion.

7. Pompe (1) selon l'une quelconque des revendications précédentes, **caractérisée en ce que** la pièce de refoulement (FE) et/ou le rotor (R) et/ou le stator (S) et/ou un moule d'appui (SG) extérieur comportant un alliage à mémoire de forme.

8. Pompe (1) selon l'une quelconque des revendications précédentes, **caractérisée en ce que** la pompe (1) comporte un système de fixation, destiné à être fixé dans le cœur et/ou dans le vaisseau.

9. Pompe (1) selon l'une quelconque des revendications précédentes, **caractérisée en ce que** dans le premier état, le stator (S) est comprimé et subit une expansion lors du transfert dans le second état.

10. Pompe (1) selon l'une quelconque des revendications précédentes, **caractérisée en ce que** dans le premier état, la pièce de refoulement (FE) n'est pas opérationnelle et devient opérationnelle par le transfert dans le second état.

11. Pompe (1) selon l'une quelconque des revendications précédentes, **caractérisée en ce qu'**à la pompe (1) est affecté un ensemble d'alimentation en énergie (EVE).

12. Pompe (1) selon la revendication 11, **caractérisée en ce que** l'ensemble d'alimentation en énergie (EVE) peut se charger par induction.

13. Pompe (1) selon la revendication 11 ou 12, **caractérisée en ce que** l'ensemble d'alimentation en énergie (EVE) peut être placé de manière extracorporelle ou intracorporelle.

14. Pompe (1) selon l'une quelconque des revendications précédentes, **caractérisée en ce qu'**à la pompe (1) est affecté un ensemble de commande (SE), qui en fonction d'un volume à refouler assiste un débit volumétrique existant.

15. Pompe (1) selon la revendication 14, **caractérisée en ce que** l'ensemble de commande (SE) peut être placé de manière extracorporelle ou intracorporelle.

16. Pompe (1) selon la revendication 14 ou 15, **caractérisée en ce que** la pompe (1) peut se commander de manière manuelle et/ou automatique.

17. Pompe (1) selon la revendication 16, dans le cas de la commande automatique, la commande étant sélectionnée dans un groupe comportant une commande programmée dans le temps, une commande hémodynamique, une commande de mouvement.

18. Pompe (1) selon l'une quelconque des revendications précédentes, **caractérisée en ce que** la pièce de refoulement (FE) présente au moins en partie la forme d'une vis ou la forme d'une hélice.

19. Pompe (1) selon l'une quelconque des revendications précédentes, **caractérisée en ce qu'**au moins une partie de la pompe (1) comporte un revêtement, destiné à réduire la friction, l'incroissance, les dépôts.

20. Pompe (1) selon l'une quelconque des revendications précédentes, **caractérisée en ce que** la pompe (1) comporte un dispositif, destiné à empêcher un reflux (DIA).

21. Pompe selon l'une quelconque des revendications précédentes, **caractérisée en ce que** la pompe (1) présente un diamètre lui permettant d'être guidée à travers un cathéter de diamètre French 32 ou inférieur.
